(19) Europäisches Patentamt
European Patent Office
Office européen des brevets

(11) Veröffentlichungsnummer: **0 085 882**
**B1**

(12) # EUROPÄISCHE PATENTSCHRIFT

(45) Veröffentlichungstag der Patentschrift: **23.04.86**

(51) Int. Cl.⁴: **A 61 F 13/20**, A 61 K 9/02, A 61 L 15/00, A 61 K 31/415

(21) Anmeldenummer: **83100613.5**

(22) Anmeldetag: **25.01.83**

(54) **Antimykotische Tampons mit hoher Wirkstoff-Freisetzung.**

(30) Priorität: **06.02.82 DE 3204124**

(43) Veröffentlichungstag der Anmeldung: **17.08.83 Patentblatt 83/33**

(45) Bekanntmachung des Hinweises auf die Patenterteilung: **23.04.86 Patentblatt 86/17**

(84) Benannte Vertragsstaaten: **DE FR GB IT**

(56) Entgegenhaltungen:
**EP - A - 0 024 023**
**EP - A - 0 058 887**
**FR - A - 2 226 986**
**FR - A - 2 245 344**
**FR - A - 2 466 988**
**US - A - 4 247 552**

**Römps Chemie-Lexikon "Lösungsvermittler"**

(73) Patentinhaber: **BAYER AG, Konzernverwaltung RP Patentabteilung, D-5090 Leverkusen 1 Bayerwerk (DE)**

(72) Erfinder: **von Bittera, Miklos, Max-Scheler-Strasse 7, D-5090 Leverkusen 3 (DE)**
Erfinder: **Büchel, Karl Heinz, Prof. Dr., Dabringhausener Strasse 42, D-5093 Burscheid (DE)**
Erfinder: **Plempel, Manfred, Dr., Zwengenberger Strasse 3c, D-5657 Haan (DE)**
Erfinder: **Regel, Erik, Dipl.-Ing., Untere Bergerheide 26, D-5600 Wuppertal 1 (DE)**

**EP 0 085 882 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neuartige Formulierungen der bekannten antimykotischen Azolderivate, die eine höhere Freisetzung der Wirkstoffe aufweisen und dadurch eine Kurzzeittherapie ermöglichen.

Es wurde gefunden, dass solche Tamponformulierungen antimykotischer Azolderivate, die den Wirkstoff in imprägnierter und erst bei Körpertemperatur schmelzender bzw. gelöster Form enthalten, den Wirkstoff in höherem Masse freisetzen und dadurch eine Verkürzung der Therapiedauer auf 1 Tag ermöglichen. Dieser Effekt der höheren Wirkstoff-Freisetzung kann bis zu einer Zehnerpotenz erreichen.

Der Wirkstoff ist in den erfindungsgemässen Tampons bevorzugt in Mengen von 50 bis 300 mg, besonders bevorzugt von 100 bis 200 mg, insbesondere in einer Menge von 100 mg vorhanden.

Die Erfindung betrifft somit antimykotische, imprägnierte Tampons enthaltend als Wirkstoff

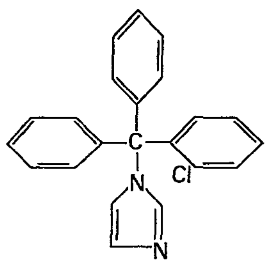

Clotrimazol

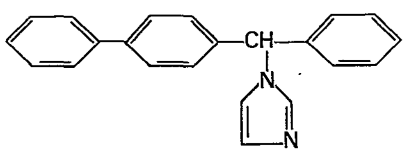

Bifonazol

oder

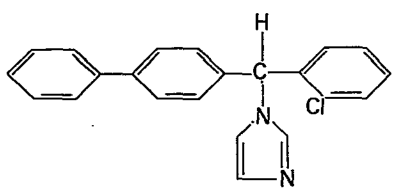

Lombazol

sowie Suppositorienmassen und/oder Spreitmittel und/oder feste Lösungsmittel, die erst bei Körpertemperatur schmelzen, dadurch gekennzeichnet, dass sie zusätzlich als Lösungsvermittler 2-Oktyldodekanol, Benzylalkohol, Ethyllactat, Propylenglykol, Di- und Tripropylenglykol und/oder ein Gemisch aus Fettsäureglyzerinpolyglykolestern, Fettsäurepolyglykolestern sowie Polyethylenglykolen und Glyzerinethoxylat, das durch Umsetzung von hydriertem Rizinusöl mit Ethylenoxid erhalten worden ist, enthalten.

Die Imprägniermasse muss folgenden Anforderungen entsprechen:

Ausreichende Konsistenz bei Raumtemperatur, Homogenität, ausreichendes Lösungsvermögen für den Wirkstoff, ausreichendes Freisetzungsvermögen, geeignetes Schmelzverhalten und gleichmässige in-vitro-Wirksamkeit.

Folgende Kombinationsmöglichkeiten sind für die Imprägnierung geeignet:

Suppositorienmassen mit Lösungsvermittlern
Suppositorienmassen mit Spreitmittel und Lösungsvermittler.

Feste, bei Körpertemperatur schmelzende Spreitmittel mit Spreitölen und Lösungsvermittler.

Feste, bei Körpertemperatur schmelzende Lösungsmittel mit zusätzlichen Lösungsvermittlern.

Für die erfindungsgemässen Mittel eignen sich folgende Suppositoriengrundlagen als Trägermaterial:

Triglycerid-Mischungen natürlicher, gesättigter Fettsäuren der Kettenlänge $C_{10}$ bis $C_{18}$, Triglyceride von Mischungen natürlicher, gesättigter, pflanzlicher Fettsäuren der Kettenlänge $C_{10}$ bis $C_{18}$, Glycerinester von Mischungen pflanzlicher, gesättigter Fettsäuren, wobei die Laurinsäure überwiegt, Suppositoriengrundlagen mit sehr niedriger Hydroxylzahl, hydroxylgruppenfreie Grundlagen. Die oben erwähnten Grundlagen mit Emulgator, z. B. nicht-ionogene Emulgatoren wie gesättigter $C_{16}$- bis $C_{18}$-Fettalkohol mit 25 Mol Ethylenoxid veräthert.

Gemische von Mono-, Di- und Triglyceriden gesättigter, natürlicher Fettsäuren der Kettenlänge $C_{12}$ bis $C_{18}$, mit oder ohne Emulgatoren.

Gemische aus Mono-, Di- und Triglyceriden, Fettalkoholen, Wachsestern.

Für die erfindungsgemässen Mittel eignen sich folgende Verbindungen als feste, bei Körpertemperatur schmelzende Spreitmittel, z. B. Myristyllactat, Cetyllactat, Myristylmyristat und ähnliche Verbindungen.

Unter Spreitmittel werden ölige Flüssigkeiten verstanden, die sich auf der Haut besonders gut verteilen (R. Keymer, Pharm. Ind. 32, 577–581 [1970]).

Für die erfindungsgemässen Mittel eigenen sich als Spreitmittel bzw. -öle insbesondere folgende Verbindungen:

Silikonöle verschiedener Viskosität.

Fettsäureester, wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$ bis $C_{18}$, Isopropylmyristat, Isopropylpalmitat, Capryl/Caprinsäureester von gesättigten Fettalkoholen der Kettenlänge $C_{12}$ bis $C_{18}$, Isopropylstearat, Ölsäureoleylester, Ölsäuredecylester, Ethyloleat, wachsartige Fettsäureester wie künstliches Entenbürzeldrüsenfett, Dibutylphthalat, Adipinsäurediisopropylester, letzterem verwandte Estergemische.

Triglyceride, wie Capryl/Caprinsäuretriglycerid, Triglyceridgemische mit Pflanzenfettsäuren der Kettenlänge $C_8$ bis $C_{12}$ oder anderen speziell ausgewählten natürlichen Fettsäuren, Partialglyceridgemische gesättigter oder ungesättigter evtl. auch hydroxylgruppenhaltiger Fettsäuren, Monoglyceride der $C_8/C_{10}$-Fettsäuren.

Fettalkohole, wie Isotridecylalkohol, Cetylstearyl-Alkohol, Oleylalkohol.

Fettsäuren, wie z. B. Ölsäure.

Besonders gut geeignete spreitende Öle sind die folgenden: Isopropylmyristat, Isopropylpalmitat, Capryl/Caprinsäureester von gesättigten Fettalkoholen der Kettenlänge $C_{12}$ bis $C_{18}$, wachsartige Fettsäureester wie künstliches Entenbürzeldrüsenfett, Silikonöl, Isopropylmyristat/Isopropylstearat/Isopropylpalmitat-Gemisch.

Glycerin, Paraffin dickflüssig, Paraffin dünnflüssig.

Für die erfindungsgemässen Mittel eignen sich folgende Mittel als Emulgatoren:

Kolloiddisperses Gemisch aus Cetyl-Stearylalkohol und Natrium-Cetyl-Stearylsulfat, Polyethylenstearat, Cetylstearylalkohol mit ca. 12 Mol Ethylenoxid, Cetylstearylalkohol mit ca. 30 Mol Ethylenoxid, Fettalkohol $C_{16}$ bis $C_{18}$ mit 25 Mol Ethylenoxid verethert, Sorbitan und Glycerinfettsäureester, ethoxyliertes Rizinusöl, Cetylstearylalkohol mit nicht-ionogenem Emulgator-Zusatz.

Folgende weitere Hilfs- und/oder Formulierungs-Grundhilfsstoffe können bei der Herstellung der erfindungsgemässen Mittel verwendet werden:

Tenside (beinhaltet Emulgatoren und Netzmittel), z.B.

1. anionaktive, wie Na-Laurylsulfat, Fettalkoholethersulfate, Mono/Dialkylpolyglykoletherorthophosphorsäureester-Monoethanolaminsalz;

2. kationaktive, wie Cetyltrimethylammoniumchlorid;

3. ampholytische, wie Di-Na-N-lauryl-β-ininodipropionat oder Lecithin;

4. nicht-ionogene, z.B. polyoxyethyliertes Rizinusöl, polyoxyethyliertes Sorbitan-Monooleat, Sorbitan-Monostearat, Cetylalkohol, Glycerinmonostearat, Polyoxyethylenstearat, Alkylphenolpolyglykolether und ähnliche Verbindungen.

Für die erfindungsgemässen Mittel eignen sich Ethylencarbonat und Polyethylenglykole mit mittleren Molgewichten als feste, erst bei Körpertemperatur schmelzende Lösungsmittel.

Als Tampon können alle handelsüblichen speziell behandelten, gepressten Baumwoll-Qualitäten verwendet werden.

Tampons mit einem Gehalt an antimykotischen Azolderivaten werden üblicherweise durch Beschichtung in folgender Weise hergestellt:

Die geschmolzene und auf 40 °C abgekühlte, aus Wirkstoff und Formulierungshilfsstoffen bestehende Grundmasse wurde in Portionen zu 2,0 g in 3 cm hohe Gläser mit einem Durchmesser von 1,5 cm eingewogen. Die vorgekühlten Tampons wurden in die Masse gedrückt, so dass sie in Höhe des Glasrandes von der erstarrenden Masse umschlossen wurden.

Die Gläser mit den Tampons wurden zum Schutz der Feuchtigkeit in Alufolien gewickelt und zur vollständigen Erstarrung in den Kühlschrank gelegt. Anschliessend liessen sich die beschichteten Tampons gut aus den Gläsern lösen.

Die erfindungsgemässen Tampons werden nach der folgenden Imprägnierungsmethode erhalten:

Die geschmolzene, auf 40 °C abgekühlte, aus Wirkstoff und Formulierungshilfsstoffen bestehende Grundmasse wurde mit einer vorgewärmten 2-ml-Injektionsspritze, deren Füllung zuvor durch Einspritzen in tarierte Tampons auf 2,0 g geeicht worden war, von der Spitze her in die noch mit Schutzfolie umwickelten vorgewärmten Tampons gespritzt.

Um ein schnelles Erstarren der Grundmasse zu verhindern, das eine gleichmässige Imprägnierung erschweren würde, wurden die imprägnierten Tampons mit der Spitze nach unten in vorgewärmte Gefässe gestellt und so langsam abgekühlt.

Das erfindungsgemässe Verfahren ermöglicht grosstechnisch eine schnelle, unkomplizierte und genauere Herstellung. Die nach diesem Verfahren imprägnierten Tampons zeigen gegenüber beschichteten Tampons eine gleichmässigere in-vitro-Wirkstoff-Freisetzung und weisen dadurch auch eine bessere therapeutische Wirksamkeit auf.

Bei den nachfolgenden Beispielen 1–39 handelt es sich um imprägnierte Zubereitungen.

Beispiel 1

| | |
|---|---|
| Clotrimazol | 200 mg/Tampon |
| Triglycerid-Mischung aus natürlichen, gesättigten Fettsäuren der Kettenlänge $C_{10}$–$C_{18}$ | 800 mg |
| Benzylalkohol | 200 mg |
| Myristylmyristat | 800 mg |

Beispiel 2

| | |
|---|---|
| Clotrimazol | 200 mg/Tampon |
| Gemisch von Mono-, Di- und Triglyceriden gesättigter Fettsäuren der Kettenlänge $C_{12}$–$C_{18}$ | 800 mg |
| Ethyllactat | 200 mg |
| Benzylalkohol | 100 mg |
| Myristylmyristat | 700 mg |

Beispiel 3

| | |
|---|---|
| Clotrimazol | 200 mg/Tampon |
| Benzylalkohol | 80 mg |
| Myristyllactat | 860 mg |
| Myristylmyristat | 860 mg |

Beispiel 4

| | |
|---|---|
| Clotrimazol | 200 mg/Tampon |
| Gemisch aus Mono-, Di- und Triglyceriden gesättigter Fettsäuren der Kettenlänge $C_{12}$–$C_{18}$ | 800 mg |
| Ethyllactat | 200 mg |
| Myristylmyristat | 800 mg |

Beispiel 5

| | |
|---|---|
| Clotrimazol | 200 mg/Tampon |
| Gemisch aus Mono-, Di- und Triglyceriden gesättigter Fettsäuren der Kettenlänge $C_{12}$–$C_{18}$ | 800 mg |
| Ethyllactat | 200 mg |
| Myristyllactat | 800 mg |

Beispiel 6

| | |
|---|---|
| Clotrimazol | 200 mg/Tampon |
| Triglycerid-Mischaung aus natürlichen, gesättigten Fettsäuren der Kettrenlänge $C_{10}$–$C_{18}$ | 800 mg |
| Ethyllactat | 200 mg |
| Benzylalkohol | 100 mg |
| Myristylmyristat | 700 mg |

Beispiel 7

| | |
|---|---|
| Clotrimazol | 200 mg/Tampon |
| Gemisch von Mono-, Di- und Triglyceriden gesättigter Fettsäuren der Kettenlänge $C_{12}$–$C_{18}$ | 1000 mg |
| Benzylalkohol | 100 mg |
| Isopropylmyristat | 100 mg |
| Myristyllactat | 400 mg |

Beispiel 8

| | |
|---|---|
| Clotrimazol | 200 mg/Tampon |
| Gemisch von Mono-, Di- und Triglyceriden gesättigter Fettsäuren der Kettenlänge $C_{12}$–$C_{18}$ | 600 mg |
| Benzylalkohol | 100 mg |
| Laurinsäurehexylester | 300 mg |
| Myristylmyristat | 800 mg |

Beispiel 9

| | |
|---|---|
| Clotrimazol | 200 mg/Tampon |
| Gemisch von Mono-, Di- und Triglyceriden gesättigter Fettsäuren der Kettenlänge $C_{12}$–$C_{18}$ | 1600 mg |
| Ethyllactat | 200 mg |

Beispiel 10

| | |
|---|---|
| Clotrimazol | 200 mg/Tampon |
| Benzylalkohol | 100 mg |
| Laurinsäurehexylester | 300 mg |
| Myristylmyristat | 1400 mg |

Beispiel 11

| | |
|---|---|
| Clotrimazol | 200 mg/Tampon |
| Benzylalkohol | 100 mg |
| Ethylencarbonat | 1700 mg |

Beispiel 12

| | |
|---|---|
| Clotrimazol | 200 mg/Tampon |
| Ethylencarbonat | 1700 mg |
| 2-Octyldodecanol | 100 mg |

Beispiel 13

| | |
|---|---|
| Clotrimazol | 200 mg/Tampon |
| Gemisch von Mono-, Di- und Triglyceriden der Kettenlänge $C_{12}$–$C_{18}$ | 844,6 mg |
| Ethyllactat | 211,0 mg |
| Benzylalkohol | 105,4 mg |
| Myristylmyristat | 739,0 mg |

Beispiel 14

| | |
|---|---|
| Clotrimazol | 100 mg/Tampon |
| Triglycerid-Mischung aus natürlichen, gesättigten Fettsäuren der Kettenlänge $C_{10}$–$C_{18}$ | 844,6 mg |
| Ethyllactat | 211,0 mg |
| Benzylalkohol | 105,4 mg |
| Myristylmyristat | 739,0 mg |

Beispiel 15

| | |
|---|---|
| Clotrimazol | 100 mg/Tampon |
| Triglycerid-Mischung aus natürlichen, gesättigten Fettsäuren der Kettenlänge $C_{10}$–$C_{18}$ | 1055,2 mg |
| Ethyllactat | 211,0 mg |
| Benzylalkohol | 105,4 mg |
| Myristyllactat | 423,0 mg |
| Isopropylmyristat | 105,4 mg |

Beispiel 16

| | |
|---|---|
| Clotrimazol | 100 mg/Tampon |
| Gemisch von Mono-, Di- und Triglyceriden gesättigter Fettsäuren der Kettenlänge $C_{12}$–$C_{18}$ | 633,4 mg |
| Ethyllactat | 211,0 mg |
| Benzylalkohol | 105,4 mg |
| Myristylmyristat | 633,4 mg |
| Laurinsäurehexylester | 316,8 mg |

Beispiel 17

| | |
|---|---|
| Clotrimazol | 100 mg/Tampon |
| Benzylalkohol | 105 mg |
| Ethylencarbonat | 1795 mg |

Beispiel 18

| | |
|---|---|
| Clotrimazol | 100 mg/Tampon |
| Polyglykol mit einem MG. von 1000 | 450 mg |
| Polyglykol mit einem MG. von 1500 | 1350 mg |
| Benzylalkohol | 100 mg |

Beispiel 19

| | |
|---|---|
| Clotrimazol | 100 mg/Tampon |
| Gemisch von Mono-, Di- und Triglyceriden gesättigter Fettsäuren der Kettenlänge $C_{12}$–$C_{18}$ | 900 mg |
| Ethyllactat | 200 mg |
| Benzylalkohol | 100 mg |
| Myristylmyristat | 700 mg |

Beispiel 20

| | |
|---|---|
| Clotrimazol | 100 mg/Tampon |
| Triglycerid-Mischung aus natürlichen, gesättigten Fettsäuren der Kettenlänge $C_{10}$–$C_{18}$ mit 2% Zusatz eines nicht-ionogenen Emulgators | 800 mg |
| Ethyllactat | 300 mg |
| Benzylalkohol | 100 mg |
| Myristylmyristat | 700 mg |

Beispiel 21

| | |
|---|---|
| Clotrimazol | 100 mg/Tampon |
| Triglycerid-Mischung aus natürlichen, gesättigten Fettsäuren der Kettenlänge $C_{10}$–$C_{18}$ | 800 mg |
| Ethyllactat | 400 mg |
| Myristylmyristat | 700 mg |

Beispiel 22

| | |
|---|---|
| Clotrimazol | 100 mg/Tampon |
| Triglyceriden gesättigter Fettsäuren der Kettenlänge $C_{12}$–$C_{18}$ | 500 mg |
| Ethyllactat | 100 mg |
| Isopropylmyristat | 200 mg |
| Myristyllactat | 400 mg |
| Myristylmyristat | 500 mg |

Beispiel 23

| | |
|---|---|
| Clotrimazol | 100 mg/Tampon |
| Triglyceriden gesättigter Fettsäuren der Kettenlänge $C_{12}$–$C_{18}$ | 800 mg |
| Ethyllactat | 400 mg |
| Benzylalkohol | 100 mg |
| Isopropylmyristat | 300 mg |
| Myristyllactat | 300 mg |

Beispiel 24

| | |
|---|---|
| Clotrimazol | 100 mg/Tampon |
| Triglyceriden gesättigter Fettsäuren der Kettenlänge $C_{12}$–$C_{18}$ | 900 mg |
| Ethyllactat | 400 mg |
| Isopropylmyristat | 300 mg |
| Myristyllactat | 300 mg |

Beispiel 25

| | |
|---|---|
| Clotrimazol | 100 mg/Tampon |
| Triglyceriden gesättigter Fettsäuren der Kettenlänge $C_{12}$–$C_{18}$ | 500 mg |
| Ethyllactat | 200 mg |
| Benzylalkohol | 100 mg |
| Laurinsäurehexylester | 200 mg |
| Myristyllactat | 400 mg |
| Myristylmyristat | 500 mg |

Beispiel 26

| | |
|---|---|
| Clotrimazol | 100 mg/Tampon |
| Triglyceriden gesättigter Fettsäuren der Kettenlänge $C_{12}$–$C_{18}$ | 900 mg |
| Ethyllactat | 200 mg |
| Benzylalkohol | 100 mg |
| Laurinsäurehexylester | 300 mg |
| Myristyllactat | 400 mg |

Beispiel 27

| | |
|---|---|
| Clotrimazol | 100 mg/Tampon |
| Triglyceriden gesättigter Fettsäuren der Kettenlänge $C_{12}$–$C_{18}$ | 900 mg |
| Ethyllactat | 400 mg |
| Laurinsäurehexylester | 300 mg |
| Myristyllactat | 300 mg |

Beispiel 28

| | |
|---|---|
| Clotrimazol | 100 mg/Tampon |
| Triglyceriden gesättigter Fettsäuren der Kettenlänge $C_{12}$–$C_{18}$ | 650 mg |
| Ethyllactat | 220 mg |
| Benzylalkohol | 110 mg |
| Laurinsäurehexylester | 320 mg |
| Myristylmyristat | 600 mg |

Beispiel 29

| | |
|---|---|
| Clotrimazol | 100 mg/Tampon |
| Triglyceriden gesättigter Fettsäuren der Kettenlänge $C_{12}$–$C_{18}$ | 800 mg |
| Triglycerid-Mischung aus natürlichen, gesättigten Fettsäuren der Kettenlänge $C_{10}$–$C_{18}$ mit 2% nicht-ionogenem Emulgator-Zusatz | 800 mg |
| Ethyllactat | 300 mg |

Beispiel 30

| | |
|---|---|
| Clotrimazol | 100 mg/Tampon |
| Polyglykol MG. 1000 | 350 mg |
| Polyglykol MG. 1500 | 1050 mg |
| Ethyllactat | 500 mg |

Beispiel 31

| | |
|---|---|
| Clotrimazol | 100 mg/Tampon |
| Polyglykol MG. 1000 | 400 mg |
| Polyglykol MG. 1500 | 11000 mg |
| Ethyllactat | 200 mg |
| Benzylalkohol | 100 mg |

Beispiel 32

| | |
|---|---|
| Clotrimazol | 100 mg/Tampon |
| Polyglykol MG. 1000 | 360 mg |
| Polyglykol MG. 1500 | 1140 mg |
| Lösungsvermittler durch Umsetzung von hydriertem Rizinusöl mit E.O. erhaltene Fettsäureglycerinpolyglykolester und Fettsäurepolyglykolester sowie Polyethylenglykole und Glycerinethoxylat | 400 mg |

Beispiel 33

| | |
|---|---|
| Clotrimazol | 100 mg/Tampon |
| Ethyllactat | 300 mg |
| Polyglykol MG. 1000 | 400 mg |
| Polyglykol MG. 1500 | 1000 mg |
| Lösungsvermittler wie in Beispiel 32 | 200 mg |

Beispiel 34

| | |
|---|---|
| Bifonazol | 100 mg/Tampon |
| Gemisch von Mono-, Di- und Triglyceriden gesättigter Fettsäuren der Kettenlänge $C_{12}$–$C_{18}$ | 900 mg |
| Ethyllactat | 400 mg |
| Laurinsäurehexylester | 300 mg |
| Myristyllactat | 300 mg |

Beispiel 35

| | |
|---|---|
| Bifonazol | 100 mg/Tampon |
| Triglycerid-Mischung aus natürlichen, gesättigten Fettsäuren der Kettenlänge $C_{10}$–$C_{18}$ mit 2% nicht-ionogenem Emulgator-Zusatz | 800 mg |
| Ethyllactat | 400 mg |
| Benzylalkohol | 100 mg |
| Myristylmyristat | 700 mg |

Beispiel 36

| | |
|---|---|
| Bifonazol | 100 mg/Tampon |
| Polyglykol MG. 1000 | 400 mg |
| Polyglykol MG. 1500 | 1000 mg |
| Ethyllactat | 200 mg |
| Benzylalkohol | 100 mg |

Beispiel 37

| | |
|---|---|
| Lombazol | 100 mg/Tampon |
| Gemisch von Mono-, Di- und Triglyceriden gesättigter Fettsäuren der Kettenlänge $C_{12}$–$C_{18}$ | 900 mg |
| Ethyllactat | 400 mg |
| Laurinsäurehexylester | 300 mg |
| Myristyllactat | 300 mg |

Beispiel 38

| | |
|---|---|
| Lombazol | 100 mg/Tampon |
| Triglycerid-Mischung aus natürlichen, gesättigten Fettsäuren der Kettenlänge $C_{10}$–$C_{18}$ mit 2% nicht-ionogenem Emulgator-Zusatz | 800 mg |
| Ethyllactat | 300 mg |
| Benzylalkohol | 100 mg |
| Myristylmyristat | 700 mg |

Beispiel 39

| | |
|---|---|
| Lombazol | 100 mg/Tampon |
| Polyglykol MG. 1000 | 400 mg |
| Polyglykol MG. 1500 | 1100 mg |
| Ethyllactat | 200 mg |
| Benzylalkohol | 100 mg |

Die Wirksamkeitstestung der erfindungsgemässen Tampons erfolgte durch Empfindlichkeitsmessung im Agardiffusionstest bei Candia albicans und Torulopsis glabrata nach folgender Methode:

Je 2 der verschiedenen, mit jeweils 100 oder 200 mg der zu prüfenden Wirkstoffe imprägnierten Vaginaltampons wurden zur Messung der Wirkstoff-Freisetzung in 10 cm hohe Bechergläser eingesetzt, die 5 cm hoch mit Kimmig-Nähragar gefüllt waren. Vor Einsetzen der Tampons wurden in die Mitte der Agarschalen mittels steriler Korkbohrer Löcher vom Durchmesser der Tampons gebohrt.

Anschliessend wurden die Agar-Oberflächen der Schalen mit Keimsuspensionen von C. albicans bzw. T. glabrata homogen beimpft. Die Keimdichte betrug $10^4$ Zellen pro $cm^2$.

Die so vorbereiteten Testschalen wurden im Brutraum bei 37 °C für 48 Stunden bebrütet.

Um ein Zeitmass für die Wirkstoff-Freisetzung aus den Vaginaltampons zu erhalten, wurde von den jeweils 2 eingesetzten Tampons je einer nach 3 Stunden, der andere nach 6 Stunden aus den Kulturschalen herausgezogen.

Nach Ende der Bebrütungszeit wurden die Hemmzonen, die sich aufgrund der Wirkstoff-Freisetzung aus den Tampons um die Testlöcher gebildet hatten, ausgemessen.

In der folgenden Tabelle sind die Hemmzonengrössen der einzelnen Tampon-Formulierungen zusammengestellt.

Tabelle

Hemmzonen-Grössen verschiedener Vaginal-Tampon-Formulierungen nach Einwirkungszeiten von 3 und 6 Std. bei C. albicans und T. glabrata.

| Formulierungs-Beispiele | Hemmzonengrösse in mm Ø nach einer Einwirkungszeit von 3 und 6 Stunden bei | | | |
|---|---|---|---|---|
| | Candida albicans | | Torulopsis glabrata | |
| | 3 Std. | 6 Std. | 3 Std. | 6 Std. |
| 1 | 36 | 36 | 30 | 34 |
| 2 | 43 | 44 | 29 | 33 |
| 3 | 37 | 38 | 29 | 31 |
| 4 | 30 | 35 | Spur | 25 |
| 5 | 32 | 33 | Spur | 26 |
| 6 | 43 | 43 | 29 | 33 |
| 7 | 45 | 45 | 30 | 34 |
| 8 | 40 | 45 | 32 | 35 |
| 9 | 30 | 39 | 24 | 27 |
| 10 | 39 | 40 | 25 | 25 |
| 11 | 40 | 40 | 38 | 41 |
| 12 | 39 | 39 | 35 | 37 |
| 13 | 40 | 41 | 33 | 35 |
| 14 | 40 | 40 | 32 | 35 |
| 15 | 35 | 36 | 27 | 28 |
| 16 | 39 | 39 | 30 | 29 |
| 17 | 39 | 39 | 37 | 38 |
| 19 | 33 | 34 | 28 | 29 |
| 20 | 34 | 39 | 28 | 28 |
| 21 | 33 | 34 | Spur | Spur |
| 22 | 32 | 36 | 30 | 30 |
| 23 | 38 | 40 | Spur | 30 |
| 24 | 34 | 35 | 26 | 27 |
| 25 | 33 | 36 | 28 | 32 |
| 26 | 34 | 34 | 29 | 30 |
| 27 | 32 | 33 | 27 | 28 |
| 28 | 31 | 32 | 28 | 28 |
| 29 | 30 | 33 | Spur | Spur |
| 30 | 32 | 37 | neg. | Spur |
| 31 | 33 | 39 | 29 | 32 |
| 32 | 34 | 43 | Spur | Spur |
| 33 | 32 | 40 | neg. | neg. |
| Bekannte Tampons (siehe Curr auf Therapeutic Research, Vol. 23, No. 6, June 1978, 661–665) | 30 | 34 | unklare Hemmzonen, zuwachsend | |

**Patentansprüche**

1. Antimykotische, imprägnierte Tampons enthaltend als Wirkstoff

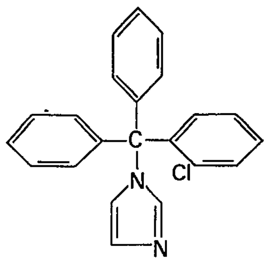

Clotrimazol

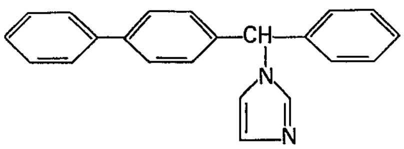

Bifonazol

oder

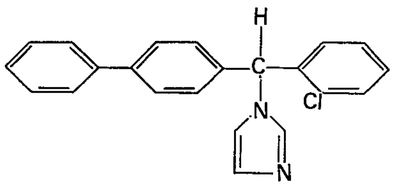

Lombazol

sowie Suppositorienmassen und/oder Spreitmittel und/oder feste Lösungsmittel, die erst bei Körpertemperatur schmelzen, dadurch gekennzeichnet, dass sie zusätzlich als Lösungsvermittler 2-Oktyldodekanol, Benzylalkohol, Ethyllactat, Propylenglykol, Di- und Tripropylenglykol und/oder ein Gemisch aus Fettsäureglyzerinpolyglykolestern und Fettsäurepolyglykolestern sowie Polyethylenglykolen und Glyzerinethoxylat, das durch Umsetzung von hydriertem Rizinusöl mit Ethylenoxid erhalten worden ist, enthalten.

2. Antimykotische Tampons gemäss Anspruch 1, dadurch gekennzeichnet, dass sie den Wirkstoff in Mengen von 50 bis 300 mg enthalten.

3. Antimykotische Tampons gemäss Anspruch 1, dadurch gekennzeichnet, dass sie den Wirkstoff in Mengen von 100 bis 200 mg enthalten.

4. Antimykotische Tampons gemäss Anspruch 1, dadurch gekennzeichnet, dass sie den Wirkstoff in einer Menge von 100 mg enthalten.

5. Verfahren zur Herstellung von Tampons gemäss Ansprüchen 1 bis 4, dadurch gekennzeichnet, dass man durch Schmelzen des Wirkstoffs und der Formulierungshilfsstoffe und anschliessende Kühlung auf 40 °C eine Grundmasse herstellt, diese Grundmasse mit einer vorgewärmten Injektionsspritze von der Spitze her in die noch mit Schutzfolie umwickelten vorgewärmten Tampons spritzt und die so imprägnierten Tampons mit der Spitze nach unten in vorgewärmte Gefässe stellt und langsam abkühlt.

**Claims**

1. Antimycotic impregnated tampons containing

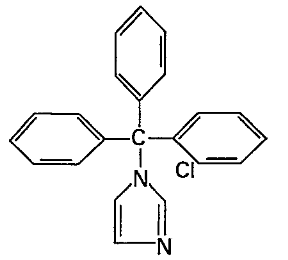

clotrimazole,

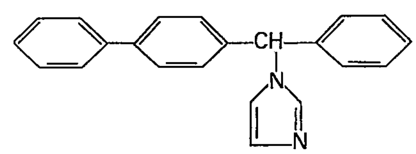

bifonazole

or

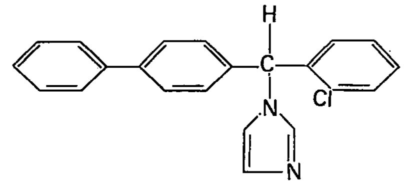

lombazole

as the active compound and suppository bases and/or spreading agents and/or solid solvents which only melt a body temperature, characterised in that they additionally contain, as solubilisers, 2-octyldodecanol, benzyl alcohol, ethyl lactate, propylene glycol, di- and tripropylene glycol and/or a mixture of fatty acid glycerol polyglycol esters, fatty acid polyglycol esters and polyethylene glycols and glycerol ethoxylate, which has been obtained by reacting hydrogenated castor oil with ethylene oxide.

2. Antimycotic tampons according to Claim 1, characterised in that they contain the active compound in quantities of 50 to 300 mg.

3. Antimycotic tampons according to Claim 1, characterised in that they contain the active compound in quantities of 100 to 200 mg.

4. Antimycotic tampons according to Claim 1, characterised in that they contain the active compound in a quantity of 100 mg.

5. Process for the production of tampons according to Claims 1 to 4, characterised in that a basic material is produced by melting the active compound and the formulating auxiliaries and subsequently cooling the melt to 40° C, this basic material is injected, by means of a prewarmed injection syringe, into the prewarmed tampons still wrapped in protective foil, via the pointed end, and the tampons thus impregnated are placed in prewarmed containers with their pointed ends at the bottom and are cooled slowly.

**Revendications**

1. Tampons antimycotiques imprégnés, contenant comme substances actives:

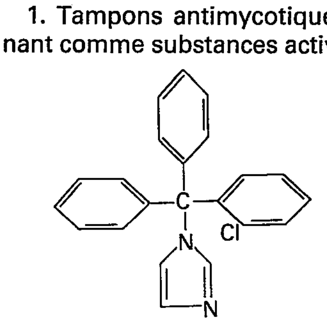

Clotrimazol

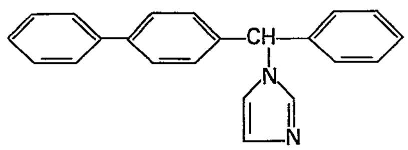

Bifonazol

ou

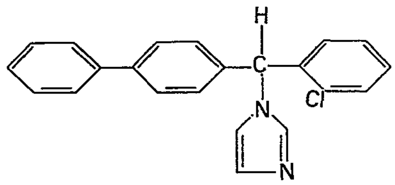

Lombazol

ainsi que des compositions pour suppositoires et/ou des agents d'étalement et/ou des solvants solides, qui ne fondant qu'à la température du corps, caractérisé en ce qu'ils contiennent en outre comme tiers-solvant, du 2-octyldodécanol, de l'alcool benzylique, du lactate d'éthyle, du propylèneglycol, du di- et tri-propylèneglycol et/ou un mélange d'esters polyglycoliques de glycérol et d'acides gras, d'esters polyglycoliques d'acides gras, ainsi que des polyéthylèneglycols et de l'éthoxylate de glycérol, que l'on a obtenu par réaction d'huile de ricin hydrogénée avec l'oxyde d'éthylène.

2. Tampons antimycotiques selon la revendication 1, caractérisés en ce qu'ils contiennent la substance active en des quantités de 50 à 300 mg.

3. Tampons antimycotiques selon la revendication 1, caractérisés en ce qu'ils contiennent la substance active en des quantités de 100 à 200 mg.

4. Tampons antimycotiques selon la revendication 1, caractérisés en ce qu'ils contiennent la substance active en une quantité de 100 mg.

5. Procédé de préparation de tampons selon les revendications 1 à 4, caractrérisé en ce qu'on prépare, par fusion de la substance active et des adjuvants de formulation puis refroidissement jusqu'à 40 °C, une composition de base, on injecte cette composition de base, à l'aide d'une seringue d'injection préchauffée, à partir de la pointe, dans les tampons préchauffés encore enveloppés d'une feuille protectrice, et l'on place les tampons ainsi imprégnés, pointe en bas, dans des récipients préchauffés et on les fait lentement refroidir.